# EUROPEAN PATENT APPLICATION

(11) **EP 2 835 454 A1**
(43) Date of publication of application: **11.02.2015**
(21) Application number: 14180000.3
(22) Date of filing: 06.08.2014
(51) Int. Cl.: C30B 33/10, C30B 29/08, C30B 29/60

(54) **Seedless group IV nanowires, and methods for the production thereof**

(30) Priority: 06.08.2013 EP 13179498
(71) Applicant: University of Limerick, Limerick (IE)
(72) Inventor: Kiely, Patrick, Limerick (IE); Ryan, Kevin, Limerick (IE); Bezuidenhout, Michael, Limerick (IE)
(74) Representative: Purdy, Hugh Barry

(57) **Abstract**

A water dispersible, biocompatible, non-toxic, seedless Group IV nanowire having an etched surface and surface oxide is provided. Also provided is a method of forming water dispersible seedless Group IV nanowires, comprising the steps of providing pristine seedless Group IV nanowires, and reacting the nanowires with a solution of an acidic amino acid to etch the nanowires and promote the formation of a water dispersible oxide layer through the formation of etched nanowires.

## Description

### Technical Field

The invention relates to seedless Group IV nanowires that are water dispersible, non-toxic to cells, and biocompatible.

### Background to the Invention

Germanium nanowires have found significant application in field emission devices, Lithium battery anodes and as antenna for light harvesting in photovoltaic cells. More recently group IV nanowires, are also finding important uses in biomedical applications ranging from single cell probing to gene delivery mechanisms. They have also proven their potential for cell adhesion platforms and in enhancing biomarker detectors. Group IV nanowires are effective in promoting hypothermia of cancer cells and silicone nanowires have also been shown to support the growth of synthetic bone coatings with potential regenerative properties for future orthopaedic applications. The use of nanowires in biological applications requires that they be non-toxic and must not adversely affect the biological activity of cells. A complication of most synthetic nanomaterials is that they can be toxic to cells. As an example, while germanium is relatively nontoxic, nanoparticles of 4.2±1.2nm have demonstrated cytotoxicity in CHO cells where low concentrations (<5µM) promote necrotic cell death. In this case alkylamine ligands required for water dispensability are toxic promoting cell damage and growth arrest whereas when the same crystal are coupled to less toxic compounds such as PEG, the negative effects are significantly reduced. Several studies performed on the surface chemistry of the nanowires have investigated the importance of the functional group as this is in direct reaction with the cellular environment. Silicon with an oxide surface functional group show less effect on biochemical processes than common hydrophilic carboxyl head groups. The orientation of anisotropic nanostructures can also directly impact the behaviour of the cellular response, for example vertically aligned wires and suspended wires differentially affect cell adhesion, cell spreading and overall cell morphology. These works highlight that any nanomaterial for biomedical use must be considered for its orientation and surface chemistry to assess the conditions which render it biocompatible. In vivo studies with GaP nanowires have been tested for their biocompatibility in rat neostriatum, in which the materials were coated with SiOₓ for its lower bioactivity interaction and thus toxicity. Such studies further highlight the importance of surface chemistry for cellular response.

While surface passivation is well studied for the device industry the approach requires using harsh chemical (HCl or HF) with subsequent Grignard or hydrogermythalation reactions offer little possibility for biocompatibility.

EP2161412 describes a method for growing nanowires by means of solution decomposition nanowires that does not employ a heavy metal seed. The method involves heating at least one high boiling point solvent to its reaction temperature in a chamber, injecting a precursor directly into the chamber to react with the at least one high boiling point solvent to produce a refluxing solvent, and subsequent vapour deposition of a monomer, achieved by the refluxing solvent, onto a locally heated substrate contained within the chamber to produce the nanowires. The seedless nanowires produced by this method are non-toxic insofar as they are not contaminated with any heavy metal, normally present in nanowires that are grown from heavy metal seeds. However, the nanowires that are produced, while non-toxic, need to be further modified to render them biocompatible.

It is an object of the invention to overcome at least one of the above-referenced problems. It is another object of the invention to provide nanowires that are non-toxic, capable of degrading in an aqueous environment, and biocompatible.

### Statements of Invention

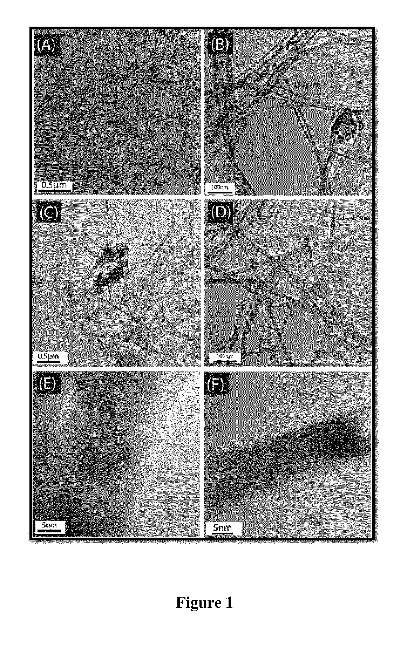

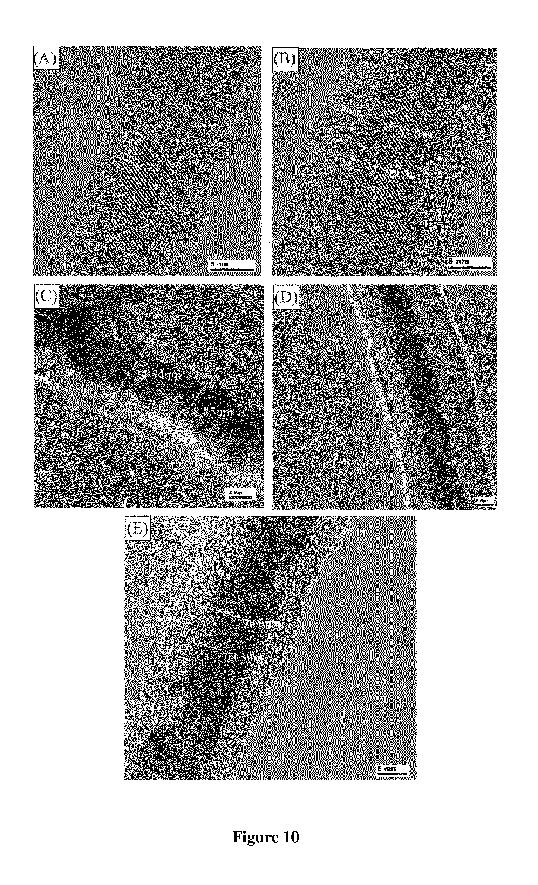

The Applicant has surprisingly discovered that seedless Group IV nanowires, when etched form a jagged saw-tooth topography (Figure 10). The subsequent wires after the native oxide forms, are water dispersible, non-toxic, and biocompatible (Figures 1 to 5). The wires have an etched surface and a thin native oxide layer (∼5nm) covering the etched surface, which unlike silicon oxide, is not stable and readily breaks down to form X(OH)₄ in aqueous (oxidising) environment (where X is a Group IV element). The Applicant has also discovered that seedless Group IV nanowires can be etched by reacting the formed nanowires with a solution of an acidic amino acid, especially glutamic acid. The data provided herein shows that the nanowires of the invention are not only non-toxic, as validated by several toxicity assays, but improve the proliferation of the two cell lines tested with this materials.

In a first aspect, the invention provides a water dispersible, biocompatible, non-toxic, seedless Group IV nanowire that is etched and comprises a native oxide coating.

In one aspect, the invention provides a water dispersible, biocompatible, non-toxic, seedless Group IV nanowire that is etched and comprises a native oxide coating, in which the Group IV element is selected from the group consisting of germanium, silicon, or derivatives thereof.

Typically, the Group IV element is germanium.

Suitably, the native oxide coating has a thickness of from 1-15nm, 1-12 nm, 1-10nm, 2-9nm, 3-8nm, 3-7nm, and ideally about 4-6nm, as determined by transmission electron microscopy. In some instances, the thickness may be from 10-12nm.

The nanowires of the invention have been found to have a native (surface) oxide coating that is non-smooth. In particular, the coating is typically a jagged saw tooth shape. This is due to the surface of the nanowire being etched, and the native (surface) oxide coating covering the etched surface. The Applicant has found that such a coated, etched surface promotes cell adhesion, thereby improving the biocompatibility of the nanowires.

Preferably, the invention relates to a biocompatible, non-toxic, germanium nanowire comprising a water dispersible native oxide that is capable of breaking down to Ge(OH)₄ in an aqueous environment.

Typically, the nanowires are capable of promoting proliferation of cells that come into contact with the nanowires.

The invention also relates to a preparation of nanowires according to the invention, for example a dispersion of the nanowires in a non-aqueous solvent.

In a second aspect, the invention provides a method of forming water dispersible seedless Group IV nanowires, especially germanium nanowires, comprising the steps of providing pristine (smooth, non-etched) seedless uncoated Group IV nanowires, and reacting the nanowires with a solution of an amino acid, preferably an acidic amino acid, to etch the nanowires and promote the formation of a native oxide coating (layer) that renders the wires water dispersible.

Preferably, the Group IV nanowires are germanium nanowires.

Preferably, the acidic amino acid is glutamic acid.

Suitably, the solution of acidic amino acid has a concentration of 0.001 to 0.01 g/L, typically 0.005 to 0.01 g/L, and ideally about 0.008 g/L.

Suitably, the pristine (smooth, non-etched) seedless uncoated Group IV nanowires are grown by solution decomposition, ideally comprising the steps of: heating at least one high boiling point solvent to its reaction temperature in a chamber, injecting a precursor directly into the chamber to react with the at least one high boiling point solvent to produce a refluxing solvent, and subsequent vapour deposition of a monomer, achieved by the refluxing solvent, onto a locally heated substrate contained within the chamber to produce the nanowires. Methods for producing pristine (smooth, non-etched) seedless uncoated Group IV nanowires are described in EP2161412, the complete contents of which are incorporated herein by reference.

In one embodiment the invention provides the step of providing a constant flow of inert argon gas coupled to said chamber to control the production of said nanowires.

In one embodiment the invention provides the step of providing a water condenser attached to the top of said chamber. Cool water flowing through the condenser restricts the reflux of the reaction to the flask. Any reflux or gas expelled from the flask is condensed to a liquid which flows back into the reaction vessel.

In one embodiment the invention provides the step of controlling the heating of the chamber at different points in the chamber to control the temperature of each reaction. The control step comprises a 3-zone heating furnace to regulate the temperature of the chamber.

In one embodiment the invention the reaction temperatures of said at least one high boiling solvent can be between 380°C and 500°C.

In one embodiment the invention the chamber comprises a lower bottom flask portion at one end and an elongated narrow channel portion at the other end.

In one embodiment the invention provides the step of producing said nanowires in said channel portion.

In one embodiment of the invention the precursor is diphenylgermane.

In one embodiment of the invention said high boiling point solvents can be one or more of the following: squalene, squalane, olelylalchol, olelylamine and/or dotriacontaine.

In one embodiment the invention provides the step of heating the substrate at a much higher temperature than the refluxing solvent, such that germane gas generated by said refluxing comes into contact with the heated substrate to decompose into germanium nanowires.

In one embodiment the invention provides germanium vapour deposition onto the substrate of said chamber nucleates the growth of the germanium nanowires.

The invention also provides water dispersible seedless Group IV nanowires formed according to a method of the invention. Preferably, the invention provides water dispersible seedless Group IV nanowires formed according to a method of the invention, in which the Group IV element is selected from the group consisting of germanium, silicon, or derivatives thereof.

The invention also provides a water dispersible seedless germanium nanowire formed according to a method of the invention.

In a further aspect, the invention provides a medical implant comprising nanowires according to the invention or nanowires formed according to a method of the invention.

In one embodiment of the medical implant, the nanowires are coated on a surface of the implant. Examples of such implants include stents, especially vascular stents, tissue engineering constructs, grafts (especially skin or tissue grafts), and bone regeneration scaffolds. The nanowires may be coated on to the surface of the implant using various techniques, including spin coating. Methods for spin-coating nanomaterials onto surfaces are described by Xia D, Biswas A, Li D, Brueck S. Directed Self-Assembly of Silica Nanoparticles into Nanometer-Scale Patterned Surfaces Using Spin-Coating. Adv. Mater. 2004;16(16):1427-1432.

In another embodiment of the medical implant, the implant of the invention is provided in the forms of a porous scaffold, especially a porous scaffold suitable for bone or tissue engineering application, in which the nanowires are dispersed within the porous scaffold. Examples of porous scaffolds for use in tissue and bone engineering applications are known from the literature, for example WO2008096334, WO2006095154, WO2006031196, EP1566186, EP1500405, WO2005004928 and EP1437148. The nanowires may be incorporated into the scaffold after scaffold manufacture, or during manufacture. For example, for scaffolds that are manufactured from a slurry that is freeze-dried, the nanowires may be incorporated into the slurry prior to freeze-drying. Such scaffolds may be made from biocompatible materials, for example collagen, fibronectin or laminin. They may also incorporate inorganic components, such as various forms of calcium phosphate (i.e. hydroxyapatite) or one or more biological growth factors (for example bone morphogenic proteins or BMPs).

In a further aspect, the invention provides a dressing or bandage for applying to a wound, in which the wound engaging surface of the dressing or bandage comprises nanowires according to the invention or nanowires formed according to a method of the invention.

The invention also relates to a pharmaceutical composition comprising nanowires according to then invention. The invention also relates to a drug-eluting implant comprising nanowires according to the invention.

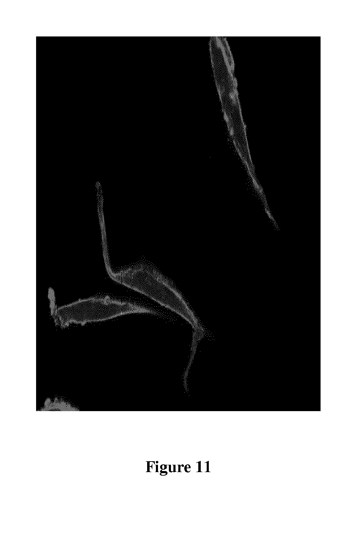

The invention also relates to a material comprising nanowires of the invention that regulates directional cell migration **(****Figure 11****).**

In this specification, the term "biocompatible" as applied to a nanowire should be understood to mean a nanowire that increases the viability of MCF-7 or L929 cells exposed to the nanowire in the MTT viability assay described below.

In this specification, the term "etched surface" as applied to a seedless Group IV nanowire should be understood to mean a nanowire in which the crystal surface of the nanowire comprises a plurality of grooves or depressions. Typically, the surface is saw tooth etched as seen in **Figure 10****.**

In this specification, the term "non-toxic" as applied to a nanowire should be understood to mean a nanowire that does not adversely affect cell membrane integrity or cellular morphology of MCF-7 or L929 cells in the cell membrane integrity and cellular morphology assays described below. In this specification, the term "seedless" as applied to a Group IV nanowire means a nanowire that does not comprise a metal seed, i.e. the synthetic method does not employ a toxic metal seed catalyst. Methods for making such seedless germanium nanowires are described in European Patent Application Publication No: 2161412 in the name of the Applicant. The nanowires are grown by solution decomposition comprising the steps of: heating at least one high boiling point solvent to its reaction temperature in a chamber, injecting a precursor directly into the chamber to react with the at least one high boiling point solvent to produce a refluxing solvent, and subsequent vapour deposition of a monomer, achieved by the refluxing solvent, onto a locally heated substrate contained within the chamber to produce the nanowires. The resultant nanowires are seedless, and therefore non-toxic, and have a smooth, ligand-free, non-etched surface. Such nanowires are also referred to as pristine nanowires. Other methods of fabricating such seedless nanowires are described by:
Zaitseva N., Harper J., Gerion D., Saw C. Unseeded growth of germanium nanowires by vapor-liquid-solid mechanism. Appl. Phys. Lett. 2005; 86(5): 053105.
Ge, M. et al. Synthesis of Germanium Nanowires. J. Phys. Chem. C. 2007; 111(30): 11157-11160.
Gerung, H., Boyle, T. J., Tribby, L. J., Bunge, S. D., Brinker, C. J., Han, S. M. Solution synthesis of germanium nanowires using a Ge2+ alkoxide precursor. J. Am. Chem. Soc. 2006; 128(15): 5244-5250.
Hobbs, R. G. et al. Seedless growth of sub-10 nm germanium nanowires. J. Am. Chem. Soc. 2010; 132(39): 13742-9.
Gerung, H., Bunge, S. D., Boyle, T. J., Brinker, C. J., Han, S. M. Anhydrous solution synthesis of germanium nanocrystals from the germanium(II) precursor Ge[N(SiMe3)2]2. Chem. Commun. (Camb) 2005; (14): 1914-6.

In this specification, the term Group IV nanowire should be understood to mean a nanowire formed of a Group IV element, namely germanium, carbon, tin, lead and silicon, or a derivative thereof.

In this specification, the term "water dispersible" as applied to the nanowires of the invention should be understood to mean that the oxide coating is unstable and the oxide coating readily break down or decompose in an aqueous (oxidising) environment, resulting in a nanowire that has greater water dispersibility than an equivalent non-etched nanowire.

In this specification, the term "non-smooth" as applied to the oxide coating on the nanowire should be understood to mean that the coating is ruffled, and is not smooth like the surface of nanowires produced according to EP2161412. The applicant has surprisingly discovered that the non-smooth surface increases the biocompatibility of the nanowires.

In this specification, the term "acidic amino acid" should be understood to mean glutamic acid, aspartic acid, and asparagine, or derivatives thereof having a similar pKa value to the parent amino acid.

### Brief Description of the Figures

**Figure 1****:** TEM image of water dispersible nanowires taken on a JEOL JEM-2010 Electron Microscope and a JEOl JEM 2100F. (A) Shows the high aspect ratio Germanium nanowires which span for micrometers. (B) Shows the complex morphology of the wires surface before treatment. (C) Shows the high aspect ratiowater dispersible Germanium nanowires which span for micrometer. (D) Show the Shows morphology of the wires after treatment. (E) Shows HRTEM of the treated wires after one week stored at 4°C in treatment solution. (F) Shows HRTEM of the pristine wires.

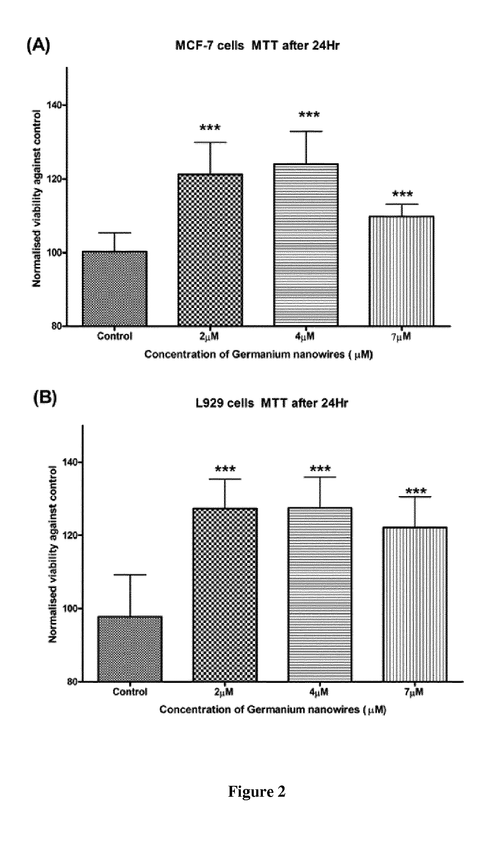

**Figure 2****:** Normalised MTT results in which cells are exposed to wire concentrations for 24 hours. (A): Shows the MCF-7 MTT results at varying concentrations over 24 hours, seeded at 27 000 cells/ well. N>3. (B): Shows the L929 MTT results at varying concentrations over 24 hours, seeded at 10 000 cells/ well N>3.Data analysis to determine the level of significance was performed using Welch's t test to determine the level of significance between treatments and control, results were considered to be significant with P<0.05. The results for the above indicate a high degree of significance ***P ≤ 0.001.

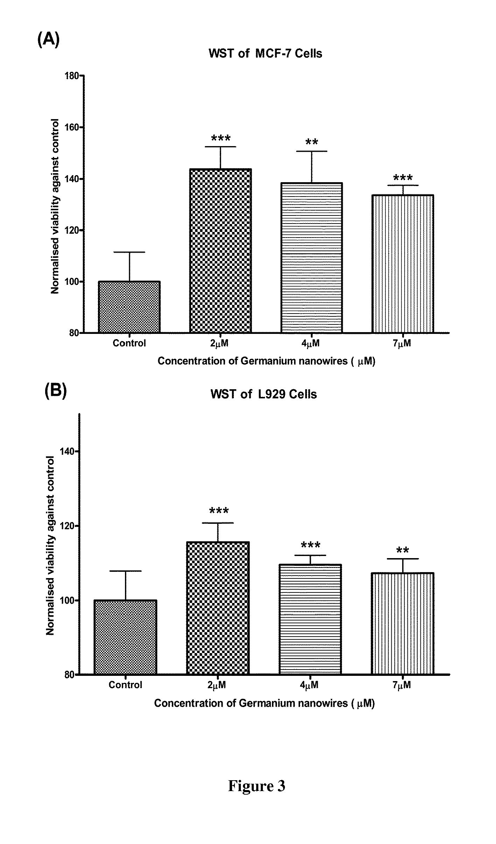

**Figure 3****:** Normalised results for the WST test. (A Normalised viability of MCF-7 cells after 24 hours of exposure to wires at varying concentrations seeded at 10 000 per well N>3. (B) Normalised WST results for L929 cells after 24 hours of exposure to wires at varying concentrations seeded at 27 000 per well N>3. Data analysis to determine the level of significance was performed using Welch's t test to determine the level of significance between treatments and control, results were considered to be significant with P<0.05. The results for the above indicate a high degree of significance ***P ≤ 0.001.

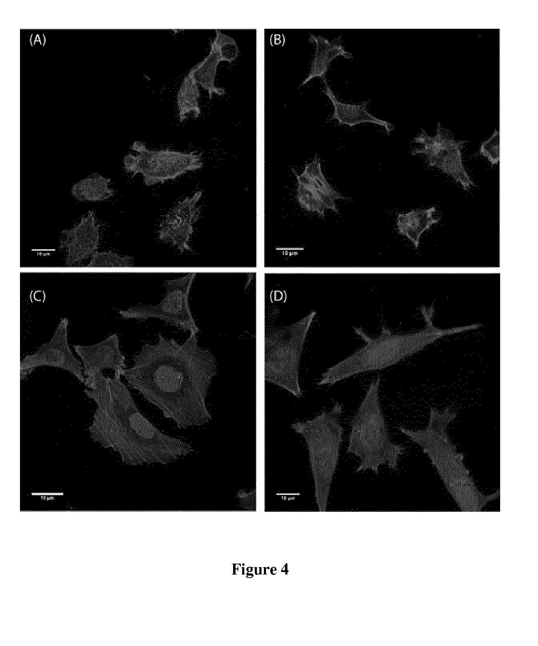

**Figure 4****:** Comparing morphology of MCF-7 and L929 cells when grown on WDW (A) L929 cell cultured on a 10µg collagen glass cover slip and stained with Hoechst DAPI and Phalloidin TRICI under a 63X oil immersion lens using a Zeiss LSM 710. Figure (B) L929 cells exposed to 4µM of Germanium nanowires for 24 hours on a 10µg collagen glass cover slip and stained with Hoechst DAPI and Phalloidin TRICI under a 63X oil immersion lens using a Zeiss LSM 710. Figure (C) MCF-7 cells cultured on a 10µg collagen coated glass cover slip and stained with Hoechst DAPI and Phalloidin TRICI under a 63X oil immersion lens using a Zeiss LSM 710. Figure (D) MCF-7 cells exposed to 4µM of Germanium nanowires for 24 hours cultured on a 10µg collagen coated glass cover slip and stained with Hoechst DAPI and Phalloidin TRICI under a 63X oil immersion lens using a Zeiss LSM 710.

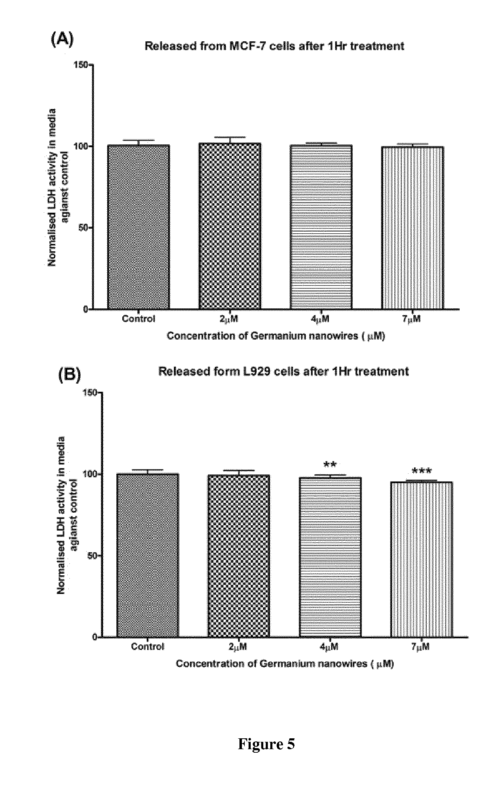

**Figure 5****:** Normalised LDH release into the serum free media results in which cells are exposed to wire concentrations for 1 hour. (A) MCF-7 cells grown on varying concentrations of WDW after 1 hour, seeded at 27 000 cells/ well. N>3. (B) L929 cells grown on varying concentrations after 1 hour, seeded at 10 000 cells/ well N>3. Data analysis to determine the level of significance was performed using one way Anova and Student-Newman-Keuls post hoc test to determine the level of significance test to determine the level of significance between treatments and control, results were considered to be significant with P<0.05. The results for the above (B) indicate a significant decrease in the LDH release into the media in which 4µM displays a mean of 97.68 ± 0.5237 N=12 **P ≤ 0.01 and 7µM displays a mean of 94.83 ± 0.3497 N=12 *P ≤ 0.5.

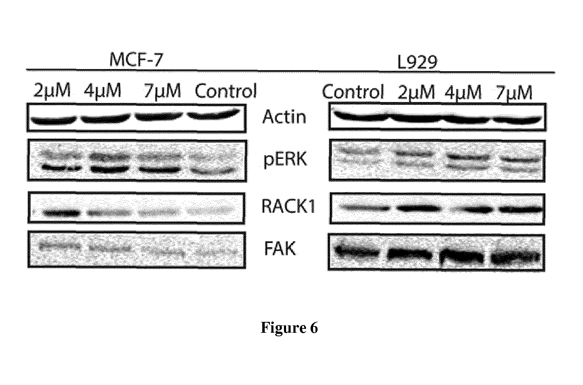

**Figure 6****:** Western immunoblotting results for MCF-7 and L929 cells. Dispersible Germanium Nanowires were added to cultures of MCF-7 and L929 cells for 24Hr before staining with Actin, RACK1 and FAK.

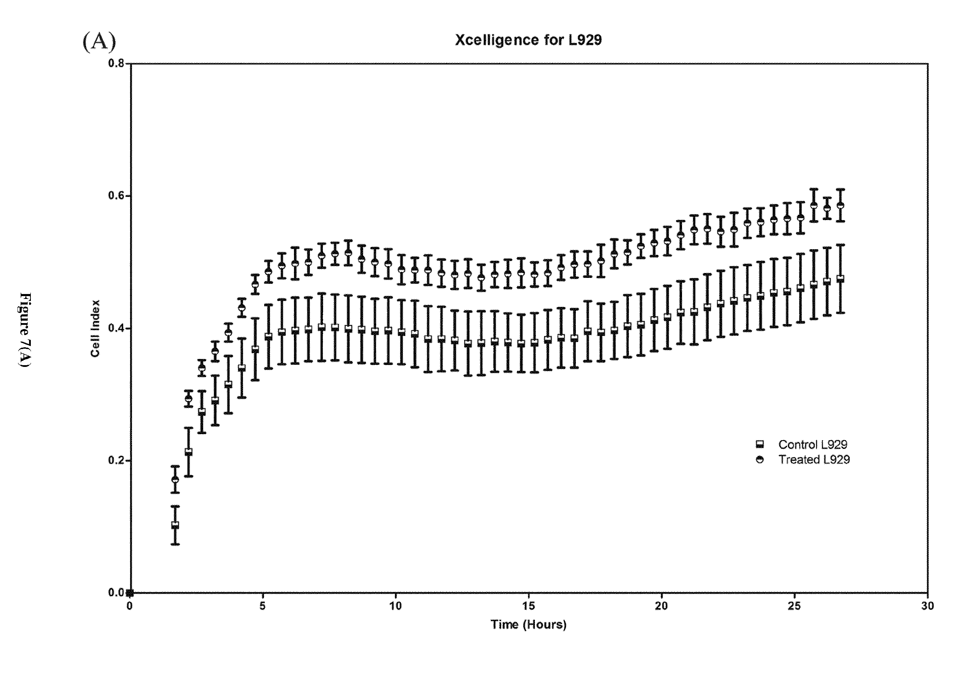

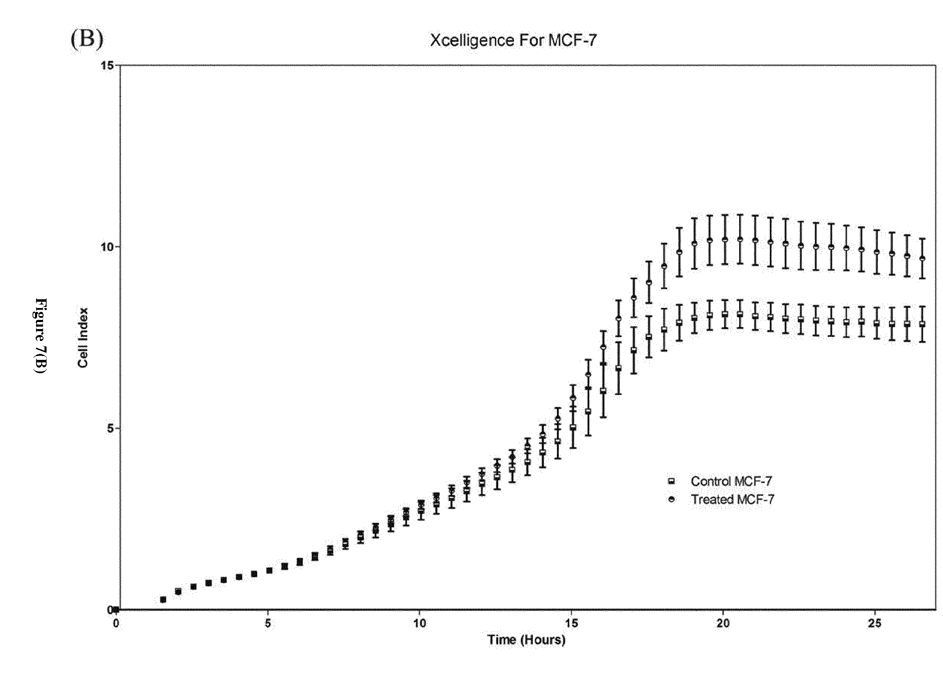

**Figure 7****:** Real time monitoring of cells grown on WDW. Cells we cultured as described for the MTT, WST and LDH assays and monitored in real time using the xCELLigence system (Roche). All experiments were carried out using E-plates following the Roche manual protocol and N=3. (A) L929 cells seeded at 10,000 cells in the presence or absence of WDW. The data indicates that there is an increase in the cell index number a measure of increased impedance across the surface of the plate. (B) MCF-7 cells seeded at 27,000 cells in the presence or absence of WDW. The data indicates that there is an increase in the cell index number a measure of increased impedance across the surface of the plate.

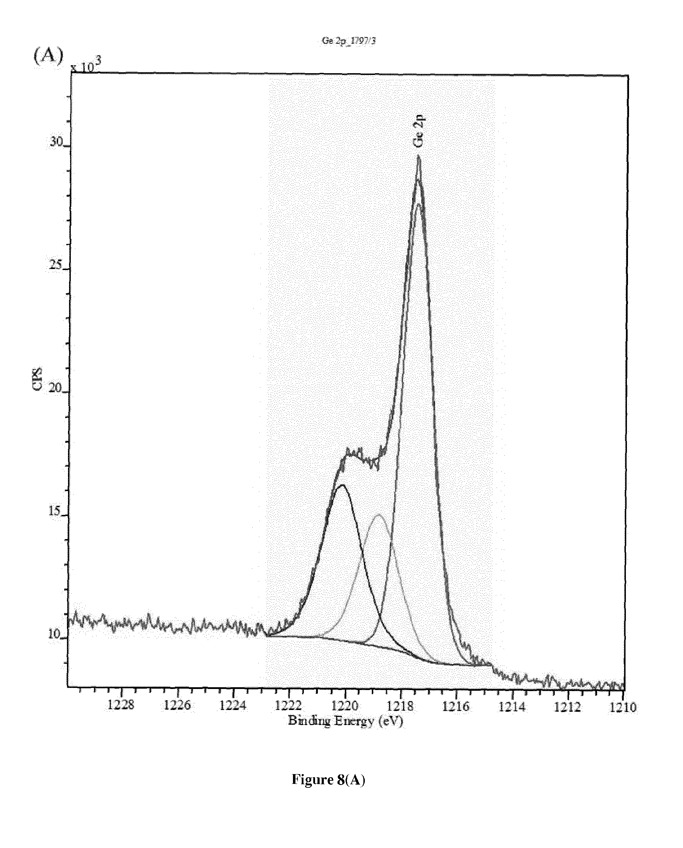

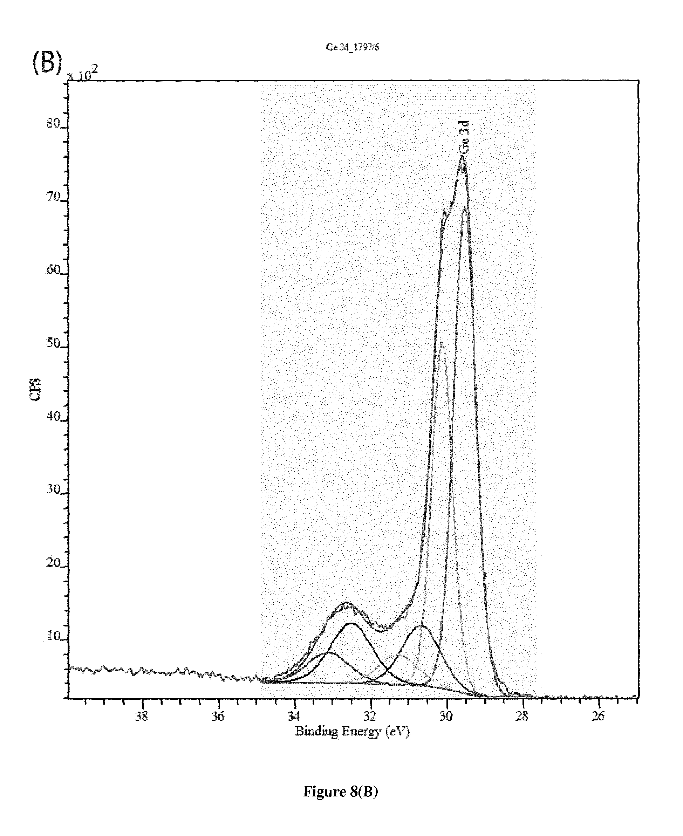

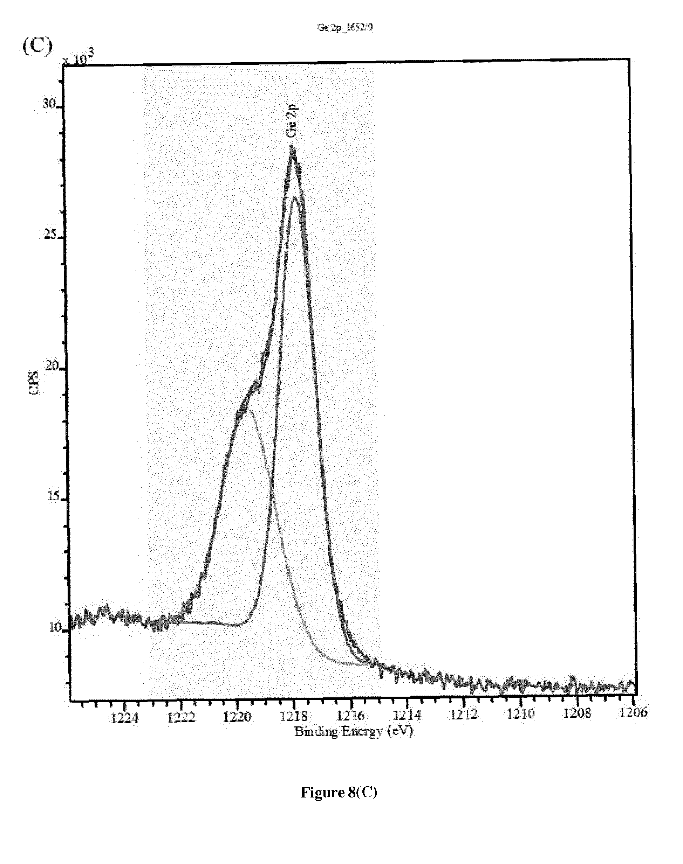

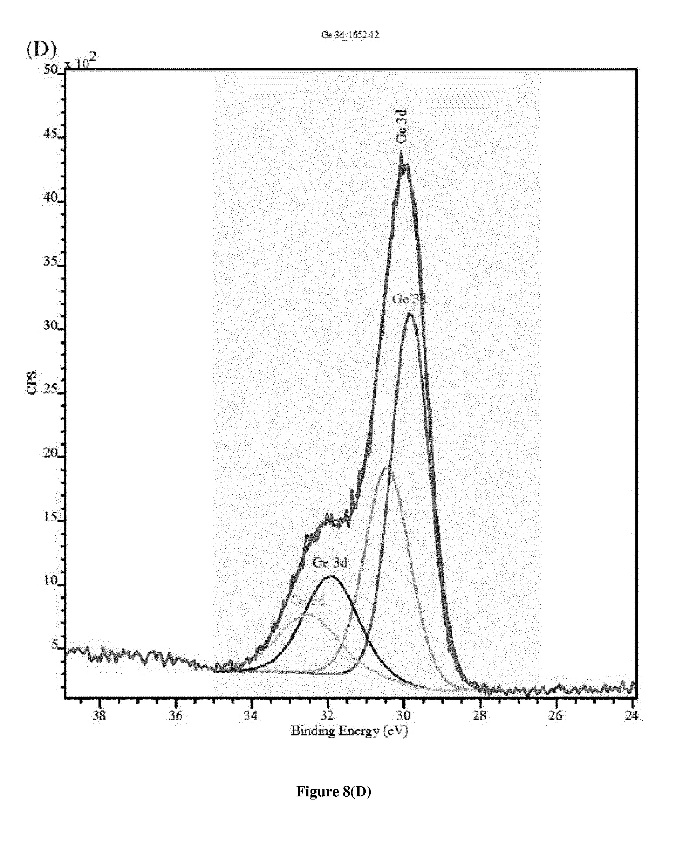

**Figure 8****:** XPS results performed on a Kratos AXIS-165. (A) The 2p binding energy associated with Germanium performed on pristine wires. (B) The 3D binding energy associated with Germanium performed on pristine wires. (C) The 2p binding energy associated with Germanium performed on treated wires. (D) The 3d binding energy associated with Germanium performed on treated wires. The data shows an increase in the relevant amount of germanium oxide in the WDW (C,D).

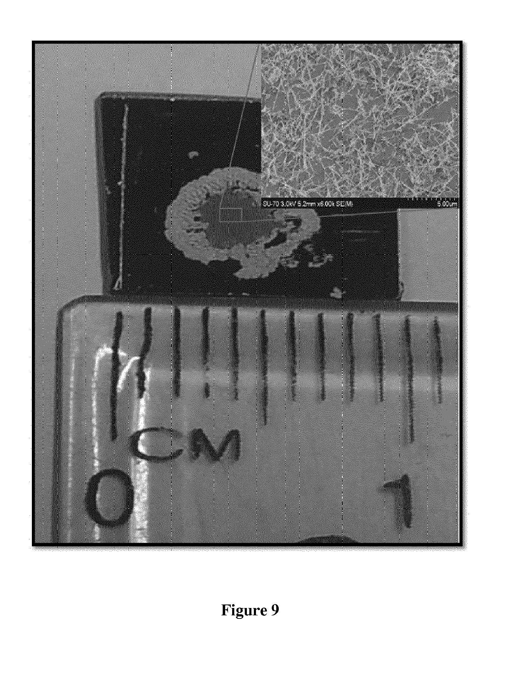

**Figure 9****:** Large scale coverage of a drop cast WDW with a representative SEM image of the area covered.
**Figure 10****:** Showing HRTEM images of water dispersible wires after different time points of etching in D-glutamic acid. (A-B) Show a nanowires with 1 Hour exposure to the Amino acid with the beginning of saw tooth etching. (C-D) Shows a nanowires with 3 hours exposure to the amino acid with prominent saw tooth etching visible. (E) Shows a nanowire with prominent saw tooth etching. All scale bars are 5nm to show scale.
**Figure 11****:** L929 mouse fibroblast cells cultured under standard conditions over a water dispersible Germanium nanowire collagen bead at 37°C @ 5% CO₂.The cells display a morphology with 90° degree growth directions imposed by the underlying Nanowire.

### Detailed Description of the Invention

### 1. Materials and methods

### 1.1 Synthesis of Germanium nanowires.

Germanium nanowires were synthesised by the high boiling point method as previously described (Barrett, C. A., et al. Perpendicular growth of catalyst-free germanium nanowire arrays. Chem. Commun. 2011. 47(13): 3843-3845). In a typical reaction Squalane (7mL, ≥99% Aldrich) is added to a long neck round bottom flask. The flask is heated under vacuum to 125°C for 1 hr to remove any residual moister. The flask is then purged with Argon and ramped to 425°C and allowed to stabilise. Diphenylgermane (DPG) (0.2mL, >95% Gelest) an organometal is then in rapidly injected into the refluxing Squalane. Reactions are allowed to run for 15min and then cooled; toluene is added to flask and then sonicated. Several washes are performed to insure the purity of the wires before samples are used for post treatment.

### 1.2 Post treatment and characterisation of Germanium nanowires.

Pristine wires suspended in toluene are centrifuged in weighed glass vials at Beckman Coulter microfuge 22R{for 20min to form a pellet. Excess toluene is removed and the wires are left at room temperature over 24hrs to remove any excess toluene. Vials are then reweighed and total nanowire mass is calculated since the surface is ligand free this is the true mass of the wires. Samples are then sterilized under UV for 40min, before adding D-Glutamic acid (0.008g/L, ≥99% Sigma) sterile filtered through a 0.22µm disposable filter. Germanium nanowires are then sonicated for 5min in EMAG 20 HC sonicator to produce a purple/brown clouded dispersion of nanowires. The surface morphology of the nanowires was characterised using a transmission electron microscope (TEM) JEOL-2010; a scanning electron microscope (SEM) Hitachi SU-70 at 10KV; and X-ray photoelectron spectroscopy (XPS) was performed using a Kratos AXIS-165.

### 1.3 Cell culture.

MCF-7 cells (human epithelial breast adenocarcinoma) and L929 cells (murine aneuploid fibrosarcoma) were cultured in DMEM media supplemented with 10% Fetal bovine Serum FBS (Sigma), 1% L-glutamine (Sigma), 1% Penicillin streptomycin (Sigma) at 37°C in a humidified incubator of 5% CO₂ as described previously (Geaney, H. et al. Role of defects and growth directions in the formation of periodically twinned and kinked unseeded germanium nanowires. Crystal Growth and Design. 2011; 11(7): 3266-3272. For all experiments cells MCF-7 were seeded at 27,000 cells/well (unless indicated) in a 96 well plate and incubated with nanowires for 24hr before endpoint testing unless stated elsewhere. L929 were seeded at 10,000 cells/well in a 96 well plate and incubated with nanowires for 24hr before endpoint testing was performed. Glass coverslips were coated with nanowires at high concentrations mixed into 10ng/µL collagen (collagen type 1 C7661 Sigma).

### 1.4 MTT Assay

Cell metabolic activity was assessed using Millipore MTT (3-(4,5-dimethylthiazol-2-yl)-2,5-diphenyl tetrazolium kit (Millipore CT02). For all experiments cells MCF-7 were seeded at 27 000 cells/well (unless indicated) in a 96 well plate and L929 were seeded at 10 000 cells/well in a 96 well plate before incubating with wires and endpoint testing. Cells were exposed to nanowires at indicated concentrations before incubation for 24hr and endpoint analysis as per kit instruction. All readings were performed on an ELx808 absorbance microplate reader BioTek, wavelength of 570 nm and a reference wavelength of 630 nm. The outer most wells were excluded from testing and filled with sterile PBS to reduce the edging effect.

### 1.5 WST assay

Cell viability was recorded using the cell proliferation reagent WST-1 (Roche 11644807001), in which the tetrazoliumsalt is cleaved to form a soluble formazan. For all experiments cells MCF-7 were seeded at 27 000 cells/well (unless indicated) in a 96 well plate and L929 were seeded at 10 000 cells/well in a 96 well plate. Cells were exposed to nanowires for 24 Hr and endpoint analysis was performed as per kit instructions all readings were performed on a Thermo scientific multiskan FC microplate photometer, using wavelengths between 420-480nm with a reference of 600nm. The outer most wells were excluded from testing and filled with sterile PBS to reduce the edging effect.

### 1.6 Western blotting and Immunoprecipitation

Cells were cultured on 10cm dishes, with a typical seeding of 1.5 million/plate. Nanowires were either coated over the plate surface or suspended into the culture media and allowed to precipitate onto the cells. Cells were cultured for 24hr before lysing by scraping into lysis buffer (20mm Tris, 50mm NaCl, 50mM NaF, 1% Npa O, Aprotinin 0.15U/ml, Leueptin 20mM, PepstatinA µ1g/ml, PMSF 2mM, Na3Vo4 0.5mM), incubating on ice for 10 min before being centrifuged for 15min at 14,000 rpm on a Beckman coulter microfuge 22R centrifuge. The protein concentration of the samples was determined using the Bradford assay. Equal protein amounts were resolved on a 12% SDS polyacrylamide gels before being transferred to a nitrocellulose membrane. The membrane was then blocked for 1Hr at room tempratue in Tris-buffered saline with 0.05% Tween 20 (TBS-T) and 5% milk (W/V). The primary antibodies were all incubated overnight at 4°C. The primary antibodies used were pERK (Cell Signaling Tec.), actin (Santa Cruza Biological,inc), RACK1 (Santa Cruza Biological,inc), FAK (Santa Cruza Biological,inc), Secondary antibodies were incubated at room temperature for 1Hr, the use of Alexa Flour 680nm and 800nm coupled anti-rabbit and anti-mouse antibodies were used for detection (Li-COR biosciences Cambridge, UK). Western blot analysis was performed on an ODYSSEY inferred imaging system (Li-COR Biosciences,Cambridge,UK).

### 1.7 Fluorescent imaging

Glass cover slips were autoclaved and placed using sterile tweezers into 24 well culturing dishes (Sigma-Corning® Costar®). MCF-7 and L929 cells were added to the wells (50,000 cells/well) and cultured as described above in the presence or absence of nanowires. After 24 hours, the cells were moved onto ice were they were washed 3 times with PHEM 1X (10mM EGTA, 25mM HERPS, 2mM MgCl₂, 60mM PIPES and adjusted using NaOH to 6.9) before fixing over night at 4°C with 3.7% PFA. Samples are then rinsed with 1X PHEM 3 times before permalising with 0.1% Triton-X in PHEM for 15min. Samples are then blocked with 5% Goat serum (Sigma) for 30min and then treated with primary antibody for 2 hours and then rinsed 3 times with PHEM. Secondary antibodies and dyes were then added for 2 hours before the coverslips were mounted onto a microscope slide and securing with vinyl (Sigma). Con-focal fluorescent imaging was performed using a Zeiss LSM 710 and processed using ZEN lite software.

### 1.8 Real-time Cell monitoring.

Continuous cell monitoring was performed using the xCELLigence system (Roche) which facilitates label free real-time cell analysis by measuring impedance-based signals [24]. Using E-plates, 100µL of complete media DMEM was added to each well and the electrodes were allowed to stabilise for 30 minutes. The plates were then moved into the xCELLigence DP analyzer to set a base line without cells or treatments. MCF-7 cells and L929 cells were then added at relevant seeding numbers (see figure legends) to the plate with varying water dispersible germanium nanowires concentrations. Cells on the electrodes were monitored by reading and recording impedance every 30 min over 100 sweeps.

### 1.9 LDH assay.

96 well plates were cultures with MCF-7 and L929 cells at 27,000 and 10,000 cells per well respectively. Cells were allowed to adhere to the plate over 8 hours before the media was change to serum free media to reduce test interference. The membrane integrity was evaluated in the presence or absence of water dispersible nanowires. We used LDH test kits (TOX7 Sigma-Aldrich) and measured membrane integrity by detecting LDH release into serum free media at 1 hour and 24 hour +/- exposure of the cells to nanowires.

### 1.10 Statistical analysis.

Data presented is expressed as mean±SD. The statistical significance between groups (concentrations and controls) was evaluated using welch t-test and for multiple groups one-way Anova was performed with post hoc test Newmans-Keuls multiple comparison test. All assumptions were tested before performing the statistical tests to insure test significance and compliance.

### 2. Results

### 2.1 Generation and characterisation of water dispersible germanium nanowires

The growth characteristics of germanium nanowires have been well documented by: Barrett, C. A. et al. Perpendicular growth of catalyst-free germanium nanowire arrays. Chem. Comm. 2011; 47(13): 3843-3845.

Geaney, H. et al. High density germanium nanowire growth directly from copper foil by self-induced solid seeding. Chemistry of Materials 2011; 23(21): 4838-4843.

Geaney, H. et al. Role of defects and growth directions in the formation of periodically twinned and kinked unseeded germanium nanowires. Crystal Growth and Design 2011; 11(7): 3266-3272.

Without treatment, the material remains hydrophobic. To achieve water dispensability, we took synthesised wires and treated them with cell friendly amino acids. Upon treatment the wires displayed good dispensability in aqueous solutions. TEM analysis of the pristine wires and the water dispersible Ge nanowires (WDW) are presented in **Figure 1****.** Pristine wires display high aspect ratio **(****Figure 1A****)** which is comparable to the WDW seen in **Figure 1C****.** This highlights the successful transfer of the material into the aqueous solution. Closer examination of the pristine wires reveals that the wires surface is relatively smooth **(****Figure 1B****).** In comparison, WDW display a rougher surface, most likely due to increase in surface oxide **(****Figure 1D****).** The HRTEM images of the WDW show crystalline material with an amorphous oxide layer **(Figure IE),** similarly pristine wires display crystalline wires with a thin amorphous oxide **(****Figure 1F****).** The dispensability of the WDW in aqueous can cover an extensive area with little aggregation unlike that of the pristine wires **(****Figure 9****).** Further surface analysis of the pristine wires show relatively low surface oxide when compared to WDW **(****Figure 8****).** Through the combination of amino acids and pristine Germanium nanowires, we are able to generate WDW with increased dispersion over larger areas which are ideal for 2 dimensional cell culturing platforms where cell coverage and contact to the material is key to the biocompatible function. The relationship between the nature of surface chemistry of a nanowire and the cellular response has been highlighted throughout literature. Work on silicon nanowires functionalised with different functional groups showed the lowest biological interference with an oxide state. We have demonstrated that our WDW have a thick oxide and thus would expect a similar decrease in cellular reaction interference as those reported.

### 2.2 Water dispersible Germanium nanowires are non toxic to MCF-7 & L929 cells.

Cell viability is an important criterion for biocompatibility and many nanomaterials have displayed cytotoxic affects under cultured condition. Previous work with Germanium nanoparticles used concentration ranges spanning 0-5µM which we used as the basis for our work. To analyse cell viability when grown on germanium nanowires, MTT assays were performed with over 20 replicates for each concentration and repeated independently (n=3+) using several different stocks of raw chemical precursors and different cell passages to insure we obtained several sets of independent results. MTT viability results are displayed with respect to the control and are based on detection of an increase in formazan production which correlates with shifts in cell viability. When grown on WDW, MCF-7 cells (human breast carcinoma cells) show an increase in viability above the control suggesting the WDW are providing advantageous conditions for the treated cells over the untreated control **(****Figure 2A****).** L929 cells (murine fibroblasts) show comparable results with that of the treated MCF-7 cells **(****Figure 2B****)** and the increase in cell viability for low concentrations of WDW is constant across the two cell lines. Data for the two cell lines when evaluated using the Welch t-test with treatments vs. the control have a ***P-value <0.0001 (n<3) showing a significant increase in the viability over the control for treatment **(****Figure 2****).** Although the MTT is considered to be the state-of-the-art standard in viability assays, we wanted to test our findings in a series of other well established techniques to validate the results beyond the MTT. To do this we employed the WST-1 test a colorimetric measurement of cell viability and proliferation which is based on reduction of WST-1 by mitochondrial succinate dehydrogenase to a coloured formazan product which reflects changes in cell viability. Both MCF- cells (Figure 3A) and L929 cells (Figure 3B) display an increase in viability above the control suggesting the WDW providing advantageous conditions for the treated cells over the untreated control. Data for the two cell lines when evaluated using the Welch t-test with treatments vs. the control have a ***P-value <0.0001(n<3), **P-value<0.01(n<3) showing a significant increase in the viability over the control for treatment (Figure 3). This increase in viability shows promise towards biocompatible nanomaterials for regenerative applications.

### 2.3 Water dispersible Germanium nanowires do not adversely affect cell membrane integrity or cellular morphology.

Cellular morphology and cell membrane integrity are an important indicator in cytotoxicity and a good indicator of cell health. Studies have shown a decrease in cell adhesion and adverse changes in cell morphology when cells are cultured with silicon nanowires. A series of studies were performed using con-focal microscopy to study the morphological features of the cells exposed to WDW's for 24 hours when compared to control cultures. MCF-7 and L929 cells were cultured on glass cover slip coated with 10µg of collagen 1 in the presence or absence of WDW's. After 24Hr cells were fixed and prepped for staining (see Materials and Methods) with phalloidin (TRICI) and Hoechst (DAPI) to highlight the Actin and nucleolus respectively. As expected, both L929 cells (Figure 4A) and MCF-7 cells (Figure 4C) and display a healthy morphology with strong focal adhesion formation to the collagen substrate. When L929 cells (Figure 4B) and MCF-7 cells (Figure 4D) were cultured with WDW, the cells show a very similar morphology to control and we observe strong focal adhesion formation with more contact points to the collagen/WDW substrate. Results shown are representative of hundreds of cells examined (n>5). It is plausible that the WDW advantageous affect may directly impact on the cellular adhesion. The con-focal microscopy supports the MTT and WST-1 results and strongly suggests that the WDW's are not toxic to the cells and provide a biocompatible surface material. These results are encouraging for biomaterial applications for promoting cell growth and adhesion.

With many high aspect ratio materials there is always concern with membrane damage. To test this, we carried out LDH release assays to evaluate the membranes integrity by recording the LDH activity present in the serum free media after exposure to the WDW for 1 hour. LDH results for both MCF-7 **(****Figure 5A****)** and L929 cells **(****Figure 5B****)** are displayed relative to the control with increases in LDH activity above the control is indicative of increase in membrane damage and LDH activity below the control is indicative of lower membrane leakage. LDH results for MCF-7 cells Figure 5(A) show no significant difference between the treated samples or that of the controls. However in L929 cells Figure 5(B) there is significant drops in the LDH released into the media for concentrations 4µM **P-value<0.01(n=12) and 7µM ***P-value <0.0001 (n=12) but this is not significant for 2 µM. The data suggest that there is no overall increase in membrane damage after initial exposure with some evidence suggesting that there the WDW may actually protect and insulate cells from membrane damage associated with routine culture.

### 2.4 Water dispersible Germanium nanowires promote the expression of proteins associated with increased cell proliferation and cell adhesion.

To investigate the changes inside the cell as a result of culturing cells on WDW, we examined the protein expression levels of FAK, and RACK1, two proteins associated with Integrin signalling pathways

Geaney, H. et al. Role of defects and growth directions in the formation of periodically twinned and kinked unseeded germanium nanowires. Crystal Growth and Design 2011; 11(7): 3266-3272.
and we measured the phosphorylation of ERK1/2, which is associated with increased proliferation. To do this MCF-7 and L929 cells were grown on a 10cm plate in the presence of wires at different concentrations (see Materials and Methods) and cultured in 10% serum for 24 hours. After this time, the cells were lysed, the protein concentration measured by Bradford assay and equivalent amounts were run on a 12% acrylamide gel before being transferred to a nitrocellulose membrane. The membrane was probed for FAK, RACK1, pERK (p42/44) and Actin as a control to confirm equivalent amounts of protein were run on the gel. As can be seen in **(****Figure 6****),** RACK1 and FAK were both expressed at higher levels when the cells were cultured on WDW. Higher expression of these proteins is associated with increased adhesion and increased proliferation. RACK1 also has an important role to play in transcription and protein synthesis (reference). The protein ERK1/2 is a member of the MAPKs (Mitogen activated protein Kinase) family which is involved with several biological process and the phosphotylation status of this protein (pERK) is a good indicator of cell proliferation. Our results for both MCF-7 and L929 cells indicate an increase in pERK with respect to the control cells, which were not treated with WDW. Based on these studies alone, the increase in pERK cannot be exclusively linked to a given pathway, however, based on our MTT and WST results **(****Figure 2** **and** **Figure 3****)** it is reasonable to assume that the increase is associated with increased proliferation of the cells.

### 2.5 Real time monitoring of cell behaviour on Water dispersible Germanium nanowires.

We have provided comprehensive evidence that WDW provide a favourable material for cell culture. The WDW do not alter the morphology of the cells and they do not damage cell membranes. Finally, we wanted to investigate the changes in the cells behaviour as they are grown on WDW using real-time measurements. To execute this, we employed the xCELLigence system for label-free and real-time monitoring of cell viability. This system measures cell adhesion, cell spreading and cell proliferation by recording cell movements across gold electrodes and correlates increased impedance as cell index. Exactly 27,000 cells/well were used for MCF-7 and 10,000 cells/well were used for L929 cells (cell counts were based on growth curves, data not shown). Samples were run in triplicates and included wells with cells +/- WDW and wells with WDW alone, to control for any effects the wires alone might have on the electrode **(****Figure 7****).** Results for both MCF-7 cells **(****Figure 7A****)** and L929 cells **(****Figure 7B****)** were plotted shows as the mean cell index ± SEM over a 27 hour period. MCF-7 cells grown with WDW deviate quickly from the control group with a steeper slop, and at 18Hrs there is clearly two distinct populations. This data strongly suggests that the WDW promotes greater cell adhesion and cell proliferation across the surface of the electrode. The L929 cells quickly adhere to the electrode and spread more rapidly than the MCF-7 cells (Figure 7B). Similarly to the MCF-7 cells, there in a dramatic increase in cell index when the L929 cells are grown with WDW. Taken together, our results provide strong evidence that WDW are a novel, non-toxic substrate. Our experiments indicate that this material is biocompatible and shows promise as a material for regenerative applications as it promotes cell adhesion and cell proliferation.

### Advantages of the Invention

The invention provides a water dispersible non-toxic Group IV nanowire having an etched surface coated with an oxide layer that makes the surface biocompatible and the nanowire water dispersible. The surface is not only non-toxic, it also promotes epithelial and fibroblast cell adhesion and cell proliferation and can be used to promote directional cell migration.

The water dispersible nanowire of the invention lends itself to use in several fields ranging from regenerative medicine to cellular physiology and cellular biophysics, drug delivery, hyperthermia induced cytotoxicity and biosensor applications.

The nanowire of the invention is suitable for development into a nanomaterial in combination with one or more extracellular matrix proteins to provide a 3-dimensional macroporous material that facilitates cell adhesion and cell proliferation.

Unlike other nanomaterials, the nanowires of the invention do not adversely affect cell membrane integrity.

The invention is not limited to the embodiments hereinbefore described which may be varied in construction and detail without departing from the spirit of the invention.

## Claims

1. A water dispersible, biocompatible, non-toxic, seedless Group IV nanowire having an etched surface and a native oxide coating, in which the Group IV element is selected from the group consisting of germanium and silicon.

2. A water dispersible, biocompatible, non-toxic, seedless Group IV nanowire as claimed in Claim 1 in which the Group IV element is germanium.

3. A water dispersible, biocompatible, non-toxic, seedless Group IV nanowire as claimed in Claim 1 or 2 in which the water dispersible native oxide coating has a thickness of from 3-7nm.

4. A water dispersible, biocompatible, non-toxic, seedless Group IV nanowire as claimed in any preceding Claim in which the native oxide coating is non-smooth.

5. A method of forming water dispersible seedless Group IV nanowires, comprising the steps of providing smooth, non-etched seedless Group IV nanowires, and reacting the nanowires with a solution of an acidic amino acid to etch the nanowires and promote the formation of a water dispersible native oxide coating through the formation of etched nanowires, in which the Group IV element is selected from the group consisting of germanium and silicon.

6. A method according to Claim 5, in which the smooth, non-etched seedless Group IV nanowires are grown by solution decomposition comprising the steps of: heating at least one high boiling point solvent to its reaction temperature in a chamber, injecting a precursor directly into the chamber to react with the at least one high boiling point solvent to produce a refluxing solvent, and subsequent vapour deposition of a monomer, achieved by the refluxing solvent, onto a locally heated substrate contained within the chamber to produce the nanowires.

7. A method as claimed in Claim 5 or 6 in which the Group IV nanowires are germanium nanowires.

8. A method as claimed in any one of Claims 5 to 7 in which the acidic amino acid is glutamic acid.

9. A method as claimed in any of Claims 5 to 8 in which the solution of acidic amino acid has a concentration of 0.0001 to 0.001 g/L.

10. Water dispersible seedless Group IV nanowires formed according to a method of any of Claims 5 to 9, in which the Group IV element is selected from the group consisting of germanium and silicon.

11. A water dispersible seedless germanium nanowire formed according to a method of any of Claims 7 to 10.

12. A medical implant comprising seedless nanowires according to Claim 1 to 4 or seedless nanowires formed according to a method of Claim 5 to 9, in which the Group IV element is selected from the group consisting of germanium and silicon,.

13. A medical implant as claimed in Claim 12 in which the seedless nanowires are coated on a surface of the implant.

14. A medical implant as claimed in Claim 12 provided in the form of a porous scaffold, in which the seedless nanowires are dispersed within the porous scaffold.

15. A dressing or bandage for applying to a wound, in which a wound engaging surface of the dressing or bandage comprises seedless nanowires according to Claim 1 to 4 or seedless nanowires formed according to a method of Claim 5 to 9 in which the Group IV element is selected from the group consisting of germanium and silicon.
